# EUROPEAN PATENT APPLICATION

(11) **EP 4 309 661 A1**
(43) Date of publication of application: **24.01.2024**
(21) Application number: 21930859.0
(22) Date of filing: 19.03.2021
(51) Int. Cl.: A61K 35/68, A61N 5/10, A61P 35/00

(54) **USE OF PLASMODIUM IN PREPARATION OF ANTI-TUMOR PREPARATION FOR USE IN COMBINATION WITH RADIOTHERAPY**

(30) Priority: 16.03.2021 CN 202110281102
(71) Applicant: CAS Lamvac (Guangzhou) Biomedical Technology Co., Ltd., Guangzhou, 510555 (CN)
(72) Inventor: TAO, Zhu, Guangzhou, Guangdong 510530 (CN); DING, Wenting, Guangzhou, Guangdong 510530 (CN); CHENG, Zhipeng, Guangzhou, Guangdong 510530 (CN); FENG, Yinfang, Guangzhou, Guangdong 510530 (CN); QIN, Li, Guangzhou, Guangdong 510530 (CN); CHEN, Xiaoping, Guangzhou, Guangdong 510530 (CN)
(74) Representative: HGF
(86) International application number: PCT/CN2021/081711
(87) International publication number: WO 2022/193267

(57) **Abstract**

Provided are the use of Plasmodium in the preparation of an anti-tumor preparation for use in combination with radiotherapy and a Plasmodium anti-tumor preparation for use in combination with radiotherapy.

## Description

### TECHNICAL FIELD

The present disclosure belongs to the field of use of biomedicine and specifically relates to use of *Plasmodium* in the preparation of an anti-tumor preparation for use in combination with radiotherapy.

### BACKGROUND

The treatment methods for tumors include three traditional modes including surgery, radiotherapy and chemotherapy (including molecular targeted therapy), and also include new biological therapies (including immunotherapy). In the clinical practice, comprehensive treatment is the mainstream. Surgery in combination with radiotherapy has a great therapeutic effect on tumors discovered in early stages, but the therapeutic effect of such a combination therapy is poor on tumors that have developed to advanced stages. Nowadays, for the treatment of advanced malignant solid tumors, various biological therapies provide more options, and molecularly targeted drugs, antibodies, immunotherapy drugs (such as PD-1) and cell therapies (such as CAR-T, TIL and CIK) have made some progress but have not been able to stop the rapid development of cancers.

Malaria is an insect-borne infectious disease caused by *Plasmodium* infection and transmitted through bites of *Anopheles* mosquitoes and has been classified by World Health Organization as one of the three worldwide infectious diseases (AIDS, tuberculosis and malaria). Five species of *Plasmodium* that can infect humans mainly include: *Plasmodium falciparum, Plasmodium vivax, Plasmodium malariae, Plasmodium ovale* and *Plasmodium knowlesi,* among which *Plasmodium falciparum* and *Plasmodium vivax* are the most common ones. *Plasmodium* that infects mice mainly includes four species: *Plasmodium chabaudi, Plasmodium yoelii, Plasmodium berghei* and *Plasmodium vinckei.*

*Plasmodium* is the pathogen of malaria. In 1981, Greentree LG first proposed the idea of malaria therapy for the treatment of tumors. In 1988, Angsubhakorn et al., scholars from Thailand, found that rats infected with *Plasmodium berghei* could prevent aflatoxin-induced liver cancer. In 2006, Liu Yingjie et al. observed in the mouse model that *Plasmodium yoelii* had a certain tumor suppression effect on synovial sarcoma cells (S180). Chen Xiaoping et al. found in the mouse model that *Plasmodium* infection could significantly inhibit the growth and metastasis of a variety of tumors such as lung cancers, liver cancers, colon cancers and breast cancers and prolong the survival of tumor-bearing mice. *Plasmodium* infection can enhance local and systemic anti-tumor specific immune responses to tumors and induce the generation of systemic long-lasting tumor-specific immune memory. *Plasmodium* infection can also improve the tumor microenvironment by significantly down-regulating the ratio or number of various immunosuppressive cells such as myeloid-derived suppressor cells (MDSCs), regulatory T cells (Tregs) and tumor-associated macrophages (TAMs) in tumor tissues, thereby releasing the immunosuppressive microenvironment in the tumor tissues and promoting the infiltration of T cells to effectively kill tumor cells.

However, not all tumor patients can generate anti-tumor responses from new immunotherapies, the high individual difference among immunotherapies remains a great hindrance in the field of tumor treatment, and there is an urgent need for strategy improvement. *Plasmodium* immunotherapy can reduce clinical toxic and side effects by controlling the infection rate through antimalarial drugs and thus has become a safe immunotherapy. Therefore, it is of great significance to develop a new *Plasmodium-based* combination therapy strategy that can achieve remarkable anti-tumor effects, which also provides a new idea for tumor treatment.

### SUMMARY

To solve deficiencies in the prior art, an object of the present disclosure is to provide use of *Plasmodium* in the preparation of an anti-tumor preparation for use in combination with radiotherapy. The present disclosure successfully combines *Plasmodium* immunotherapy (a biological therapy) with radiotherapy, and such a combination therapy has a high-level biosafety, has a more significant inhibition effect on the growth of tumors than single *Plasmodium* immunotherapy or single radiotherapy, prolongs the survival of tumor-bearing mice, and provides a new strategy and an idea for the treatment of tumors. The *Plasmodium* immunotherapy increases the ratio or absolute number of CD4⁺ T cells and CD8⁺ T cells generated in the peripheral blood and the spleen of the body through infection, reduces the ratio of Tregs and MDSCs in the peripheral blood and the spleen, to inhibit the tumor growth. The radiotherapy reduces tumor burden by directly killing tumor cells, but the radiotherapy reduces the absolute number of CD4⁺ T cells and CD8⁺ T cells in the spleen, and damages a certain anti-tumor immunity. Both the *Plasmodium* immunotherapy and the radiotherapy can promote the infiltration of immune cells and exert complementary and synergistic anti-tumor effects.

To achieve the object, the present disclosure adopts the technical solutions described below.

In a first aspect, the present disclosure provides use of *Plasmodium* in the preparation of an anti-tumor preparation for use in combination with radiotherapy.

Due to the complexity of the cause of tumors, a single immunotherapy or a single radiotherapy has greater limitations in treating tumors and fails to achieve the expected therapeutic effect. In the present disclosure, *Plasmodium* immunotherapy in combination with radiotherapy is applied as an anti-tumor preparation for the first time. The *Plasmodium* immunotherapy has a multi-target effect by fully activating the natural immune system and inducing a certain specific immunity. However, the *Plasmodium* immunotherapy cannot directly kill tumors, and the larger tumor size will affect the efficacy of the *Plasmodium* immunotherapy. The radiotherapy can directly kill tumor cells, reduce tumor burden, release tumor antigens, and facilitate the intervention of the immune system activated by the *Plasmodium immunotherapy,* thereby enhancing the anti-tumor immunity.

The *Plasmodium* infection fully activates the natural immune system, including the ratio and absolute number of CD4⁺ T cells and CD8⁺ T cells, and although the radiotherapy can directly kill tumors, the radiotherapy also damages the absolute number of lymphocytes and has a certain immunosuppression effect. The *Plasmodium* infection stimulates macrophages and dendritic cells (DCs), highly expresses MHC II, CD80, CD86 and CD28 molecules, promotes the secretion of various factors such as IL-12, IFN-α/β/γ, TNF-α, IL-8 and NO of these molecules, and promotes the differentiation and maturation of antigen-presenting cells. *Plasmodium* in the erythrocytic stage can widely activate immune cells of the host in the early stage of infection, induce the generation of a large number of cytokines such as IFN-γ and TNF-α, activate NK cells, γδ T cells and NKT cells and enhance their killing ability, thereby enhancing anti-tumor specific immune responses of the tumor local and systemic. The *Plasmodium* infection significantly inhibits angiogenesis in tumor tissues by acting on multiple pathways and targets for angiogenesis through down-regulation of TAMs and generation of large amounts of exosomes. Meanwhile, the *Plasmodium* infection can also induce the generation of a systemic long-lasting tumor-specific immune memory.

The radiotherapy directly kills tumor cells by destroying cell chromosomes. The radiotherapy also causes the ionization in the body to generate free radicals, and the free radicals interact with biomacromolecules and cause irreversible damages to cells, thereby directly and indirectly destroying tumor tissues. The tumor cells show a high degree of diversity after the radiotherapy, from apoptosis and necrosis to autophagy and mitotic catastrophe. A large number of tumor antigens are released, and protein translation is increased, thereby promoting a significant increase in the amount and type of tumor-associated antigens. These antigens are exposed on the surface of the tumor cells through calprotectin, and molecules such as HMGB 1 and ATP are released, promoting the uptake and presentation of tumor antigens by DC cells and the activation of T-cells. The *Plasmodium* infection can improve the tumor microenvironment by significantly down-regulating the ratio or number of various immunosuppressive cells such as myeloid-derived suppressor cells (MDSCs), regulatory T cells (Tregs) and tumor-associated macrophages (TAMs) in tumor tissues, thereby promoting the infiltration of T cells into tumor tissues and effectively killing tumor cells.

In summary, the *Plasmodium* infection in combination with radiotherapy increases the ratio and absolute number of CD4⁺ T cells and CD8⁺ T cells in the body, enhances the recognition of more specific antigens by antigen-presenting cells, facilitates the anti-tumor immune response of the body, effectively removes residual tumor cells, increases the anti-tumor cytokines such as IFN-γ and TNF-α in the human body, activates the NK cells, γδ T cells, and NKT cells, enhances the tumor-killing ability of these cells, reduces the ratio or number of Tregs, MDSCs and TAMs in the tumor microenvironment, and can form complementary and synergistic anti-tumor effects.

Preferably, the *Plasmodium* includes any one or a combination of at least two of *Plasmodium falciparum, Plasmodium vivax, Plasmodium malariae, Plasmodium ovale* or *Plasmodium knowlesi,* for example, a combination of *Plasmodium falciparum* and *Plasmodium vivax,* a combination of *Plasmodium vivax* and *Plasmodium malariae,* and a combination of *Plasmodium ovale* and *Plasmodium knowlesi,* and any other combination may be selected, which will not be described in detail herein.

The *Plasmodium* preferably is *Plasmodium vivax.*

Preferably, an inoculation amount of the *Plasmodium* is not less than 100 *Plasmodia* or not less than five *Plasmodium* sporozoites, for example, 100 *Plasmodia,* 120 *Plasmodia,* 140 *Plasmodia,* 150 *Plasmodia,* 160 *Plasmodia,* 5 *Plasmodium* sporozoites, 8 *Plasmodium* sporozoites, 10 *Plasmodium* sporozoites, 15 *Plasmodium* sporozoites, and any other specific value may be selected, which will not be described in detail herein.

The inoculation amount of the *Plasmodium* may be adjusted by those skilled in the art according to the actual situation. Due to the individual difference among patients, the number of the *Plasmodium* that can be infected with varies, and generally, an inoculation amount of the *Plasmodium* is not less than 100 *Plasmodia* or not less than five *Plasmodium* sporozoites.

The *Plasmodium* infection involved in the present disclosure is a long-range *Plasmodium* infection. The longer the infection cycle, the more significant the inhibition effect on the tumor. The long-range *Plasmodium* infection is a *Plasmodium* infection where *Plasmodium* lasts until the chronic stage of the *Plasmodium* infection. The long-range *Plasmodium* infection is maintained for a certain period of time, and then the infection is terminated by administrating an antimalarial drug or the infection is not terminated so that the patients are in the state of carrying the *Plasmodium.* The *Plasmodium* infection enters the chronic stage after an acute stage of the infection of about 6 to 8 weeks. At this point, only a small amount of *Plasmodium* can be detected in the peripheral blood, but no clinical symptom of the acute stage occurs.

Preferably, the radiotherapy includes an alpha ray, a beta ray, a gamma ray, an X ray, an electron beam or a proton beam.

Preferably, the radiotherapy includes a single radiotherapy, a multiple radiotherapy or a fractionation radiotherapy.

Preferably, the radiotherapy includes a systemic radiotherapy or a local radiotherapy.

Preferably, the tumor includes a lung cancer, a gastric cancer, a colon cancer, a liver cancer, a breast cancer, a prostate cancer, an ovarian cancer, a pancreatic cancer, and a brain tumor.

Preferably, a dosage form of the preparation includes any one of pharmaceutically acceptable dosage forms.

Preferably, the preparation further includes any one or a combination of at least two of pharmaceutically acceptable pharmaceutical adjuvants. The adjuvant includes any one or a combination of at least two of an excipient, a diluent, a carrier, a flavoring agent, a binder, or a filler.

Preferably, the *Plasmodium* is a *Plasmodium* carried on a pharmaceutical carrier.

Preferably, the pharmaceutical carrier includes a liposome, a micelle, a dendrimer, a microsphere or a microcapsule.

In a second aspect, the present disclosure provides an anti-tumor preparation. The anti-tumor preparation is a *Plasmodium* preparation for use in combination with radiotherapy.

The anti-tumor preparation has a high-level biosafety, has a stronger inhibition effect on the growth of tumors than single *Plasmodium* immunotherapy and single radiotherapy, can more effectively prolong the survival of tumor-bearing mice, provides a new strategy and an idea for the treatment of tumors, can reduce the dose of radiotherapy or the number of times of radiotherapy to reduce the damage of the radiotherapy to the immune system of the patients, reduces the side effects of the radiotherapy, and reduces the treatment costs for the tumor patients. The combination therapy of *Plasmodium* immunotherapy and radiotherapy produces stronger anti-tumor specific responses through complementary and synergistic effects.

For the *Plasmodium* in the anti-tumor preparation, only the *Plasmodium* or the combination of *Plasmodium* and other adjuvants needs to be inputted into the tumor patients so that the tumor patients are successfully infected with the *Plasmodium,* and those skilled in the art may choose a method known in the art to carry out the input process according to the actual situation, preferably by way of injection.

In the present disclosure, since the chronic *Plasmodium* infection formed after a person is infected with *Plasmodium* can persist for as long as several years and the person can also be repeatedly infected with the same or different species of *Plasmodium,* the state of long-term recurrent *Plasmodium* infection can be formed, and the *Plasmodium* can inhibit the growth of the tumor cells, prolong the life span of the patients, provide the tumor patients with a greater treatment opportunity and a better immune environment of the body, and facilitate the treatment and recovery of the patients with the brain tumor.

In the present disclosure, the *Plasmodium* infection can enhance the immune surveillance mechanism against tumors. In the *Plasmodium* infection process, the pathogen-associated molecular patterns (PAMPs) of danger signal molecules are released. The PAMPs including glycosylphosphatidylinositol anchors (GPI anchors), hemes, immunostimulatory nucleic acid motifs and other unknown molecules can be recognized by the pattern recognition receptors (PRRs) of the immune cells of the host. Different transcriptional programs are triggered and multiple downstream signaling pathways are stimulated to induce the generation of systemic immune responses, including the release of cytokines such as TNF-α, IL-1β, IL-2, IL-6, IL-12 and IFNs, the activation of NK cells, NKT cells, γ/δT cells and DC cells, then, the activation of the ratio or absolute number of CD4⁺ T cells and CD8⁺ T cells, the down-regulation of cytokines such as TGF-β and IL-10, and the down-regulation of the ratio of Tregs, MDSCs and TAMs by the down-regulation of cytokines such as TGF-β and IL-10, thereby improving the immunosuppressive microenvironment of the tumors. The *Plasmodium* infection induces adaptive anti-tumor immunity by increasing tumor-specific T cell proliferation and cytotoxic (CTL) activity of CD8⁺ T cells and promotes the infiltration of these cells into the tumor tissues to killing tumor cells.

In the present disclosure, the fever caused by the *Plasmodium* infection may promote the death of tumor cells. In the medical literature, the febrile infection is related to the spontaneous regression of tumors, and malaria is a typical febrile infection. For mice, the *Plasmodium* only causes short-term infection without fever, and the repeated *Plasmodium* infection is seldom observed in murine models. For humans, the *Plasmodium* infection can cause long-term parasitemia accompanied by acute-phase hyperthermia, and such a syndrome can be repeated many times throughout the lifespan. Therefore, the *Plasmodium* naturally acquired through the bites of *Anopheles* mosquitoes will cause *Plasmodium* infection of the liver phase and blood phase that continuously stimulates the immune system to convert the tumor into an effective tumor vaccine. It, together with the acute-phase fever and its tumor angiogenesis inhibition effect, exerts a multi-pathway and multi-target therapeutic effect on the tumor.

In the present disclosure, radiotherapy is to irradiate the tumor tissues of the tumor patients by rays such as alpha rays, beta rays, gamma rays, X rays, electron beams or proton beams to directly destroy the chromosomes of the cells, thereby controlling the growth of the tumor. Radiotherapy treats tumors of different types and tumors in different stages by adjusting the number of times of fractionation radiation, the dose of single radiation and total radiation dose. Radiotherapy kills the tumor locally, releases tumor antigens, and induces local tumor vaccines to cause the shrinkage or disappearance of the tumor at a distance that has not been treated by radiotherapy, occurring the "abscopal effect". Radiotherapy induces and improves anti-tumor immune effects by activating the immune system of the body.

Preferably, the preparation and the radiotherapy are administered concurrently.

Preferably, the preparation and the radiotherapy are administered sequentially.

The preparation and radiotherapy may be administered concurrently or sequentially. For example, the *Plasmodium* infection may be administered first, and then radiotherapy is administrated after some interval of time; radiotherapy may be administered first, and then the *Plasmodium* infection is administrated after some interval of time; or the *Plasmodium* infection and radiotherapy may be administered alternately.

Preferably, the *Plasmodium* includes any one or a combination of at least two of *Plasmodium falciparum, Plasmodium vivax, Plasmodium malariae, Plasmodium ovale* or *Plasmodium knowlesi,* and preferably is *Plasmodium vivax.*

Preferably, an inoculation amount of the *Plasmodium* is not less than 100 *Plasmodia* or not less than five *Plasmodium* sporozoites, for example, 100 *Plasmodia,* 120 *Plasmodia,* 140 *Plasmodia,* 150 *Plasmodia,* 160 *Plasmodia,* 5 *Plasmodium* sporozoites, 8 *Plasmodium* sporozoites, 10 *Plasmodium* sporozoites, 15 *Plasmodium* sporozoites, and any other specific value may be selected, which will not be described in detail herein.

Preferably, the tumor includes a lung cancer, a gastric cancer, a colon cancer, a liver cancer, a breast cancer, a prostate cancer, an ovarian cancer, a pancreatic cancer or a brain tumor.

Preferably, an administration manner of the preparation includes intravenous injection, intraperitoneal injection, intramuscular injection, subcutaneous injection, oral administration, sublingual administration, nasal administration or percutaneous administration.

In a third aspect, the present disclosure provides an anti-tumor combination therapy. The anti-tumor combination therapy is a combination of a radiotherapy and a *Plasmodium* therapy.

Preferably, the radiotherapy and the *Plasmodium* are administered concurrently.

Preferably, the radiotherapy and the *Plasmodium* are administered sequentially.

Preferably, the *Plasmodium* includes any one or a combination of at least two of *Plasmodium falciparum, Plasmodium vivax, Plasmodium malariae, Plasmodium ovale* or *Plasmodium knowlesi,* and preferably is *Plasmodium vivax.*

Preferably, an inoculation amount of the *Plasmodium* is not less than 100 *Plasmodia* or not less than five *Plasmodium* sporozoites.

Preferably, the tumor includes a lung cancer, a gastric cancer, a colon cancer, a liver cancer, a breast cancer, a prostate cancer, an ovarian cancer, a pancreatic cancer, and a brain tumor. Preferably, an administration manner of the *Plasmodium* includes intravenous injection, intraperitoneal injection, intramuscular injection, subcutaneous injection, oral administration, sublingual administration, nasal administration or percutaneous administration, and preferably is intravenous injection.

Compared with the prior art, the present disclosure has the beneficial effects described below.

The present disclosure successfully combines *Plasmodium* with radiotherapy, and such a combination therapy has a high-level biosafety. The *Plasmodium* mainly causes intermittent cold and heat attacks, and the infection rate can be controlled by using antimalarial drugs to avoid severe cold and heat attacks, thereby ensuring the safety and preventing the normal functions of the brain and the functions of other organs and tissues from damage. The combination therapy also has a more significant inhibition effect on the growth of tumors than single *Plasmodium* therapy or single radiotherapy, and inhibits the growth of tumors mainly through directly killing of tumors, complementary and synergistic immune effects, and enhancing specific anti-tumor responses, thereby prolonging the survival of the patients. The anti-tumor combination preparation involved in the present disclosure is relatively economical. Compared with the standard chemotherapy commonly used in the clinical practice whose cost is high, the *Plasmodium* immunotherapy is relatively much lower in cost. With the injection of *Plasmodium* sporozoites or *Plasmodium* in the erythrocytic stage into the patients, the infection rate of *Plasmodium* is controlled through low-cost antimalarial drugs, the side effects of *Plasmodium* infection are relatively few, and only simple symptomatic treatment, regular blood routine examination and monitoring of hepatorenal functions are required to be applied to the tumor patients, thereby freeing the patients from additional costs. The treatment can be repeated or terminated at any time according to the change of the condition, and the course of treatment is controllable. The combination of *Plasmodium* and radiotherapy is a relatively safe, economical and feasible new means of tumor treatment and provides a new strategy and an idea for the treatment of tumors.

### BRIEF DESCRIPTION OF DRAWINGS

FIG. 1 is a graph showing tumor growth curves of subcutaneously inoculated LLC lung cancer under the treatment of Py17XNL *Plasmodium* infection in combination with radiotherapy;
FIG. 2 is graph showing comparison results of tumor weights of subcutaneously inoculated LLC lung cancer under the treatment of Py17XNL *Plasmodium* infection in combination with radiotherapy on Day 19;
FIG. 3 is graph showing photographed pictures of tumor tissues of subcutaneously inoculated LLC lung cancer under the treatment of Py17XNL *Plasmodium* infection in combination with radiotherapy on Day 19;
FIG. 4 is a graph showing survival curves of tumor-bearing mice subcutaneously inoculated with LLC lung cancer under the treatment of Py17XNL *Plasmodium* infection in combination with radiotherapy;
FIG. 5 is a graph showing weight growth curves of tumor-bearing mice subcutaneously inoculated with LLC lung cancer under the treatment of Py17XNL *Plasmodium* infection in combination with radiotherapy;
FIG. 6 is a graph showing *Plasmodium* infection rate curves for subcutaneously inoculated LLC lung cancer under the treatment of Py17XNL *Plasmodium* infection in combination with radiotherapy;
FIG. 7 is a graph showing statistical results of the CD3+/CD45+ ratio in the peripheral blood for subcutaneously inoculated LLC lung cancer under the treatment of Py17XNL *Plasmodium* infection in combination with radiotherapy;
FIG. 8 is a graph showing statistical results of the CD8+/CD45+ ratio in the peripheral blood for subcutaneously inoculated LLC lung cancer under the treatment of Py17XNL *Plasmodium* infection in combination with radiotherapy;
FIG. 9 is a graph showing statistical results of the CD4+/CD45+ ratio in the peripheral blood for subcutaneously inoculated LLC lung cancer under the treatment of Py17XNL *Plasmodium* infection in combination with radiotherapy;
FIG. 10 is a graph showing statistical results of the Treg/CD4+ ratio in the peripheral blood for subcutaneously inoculated LLC lung cancer under the treatment of Py17XNL *Plasmodium* infection in combination with radiotherapy;
FIG. 11 is a graph showing statistical results of the MDSC/CD45+ ratio in the peripheral blood for subcutaneously inoculated LLC lung cancer under the treatment of Py17XNL *Plasmodium* infection in combination with radiotherapy;
FIG. 12 is a graph showing statistical results of the CD3+/CD45+ ratio in the spleen for subcutaneously inoculated LLC lung cancer under the treatment of Py17XNL *Plasmodium* infection in combination with radiotherapy;
FIG. 13 is a graph showing statistical results of the CD8+/CD45+ ratio in the spleen for subcutaneously inoculated LLC lung cancer under the treatment of Py17XNL *Plasmodium* infection in combination with radiotherapy;
FIG. 14 is a graph showing statistical results of the CD4+/CD45+ ratio in the spleen for subcutaneously inoculated LLC lung cancer under the treatment of Py17XNL *Plasmodium* infection in combination with radiotherapy;
FIG. 15 is a graph showing statistical results of the Treg/CD4+ ratio in the spleen for subcutaneously inoculated LLC lung cancer under the treatment of Py17XNL *Plasmodium* infection in combination with radiotherapy;
FIG. 16 is a graph showing statistical results of the MDSC/CD45+ ratio in the spleen for subcutaneously inoculated LLC lung cancer under the treatment of Py17XNL *Plasmodium* infection in combination with radiotherapy;
FIG. 17 is a graph showing statistical results of the CD8 total number in the spleen for subcutaneously inoculated LLC lung cancer under the treatment of Py17XNL *Plasmodium* infection in combination with radiotherapy;
FIG. 18 is a graph showing statistical results of the CD4 total number in the spleen for subcutaneously inoculated LLC lung cancer under the treatment of Py17XNL *Plasmodium* infection in combination with radiotherapy;
FIG. 19 is a graph showing tumor growth curves of intracranially inoculated GL261/mchery-Luc glioma under the treatment of Py17XNL *Plasmodium* infection in combination with radiotherapy;
FIG. 20 is a graph showing *in vivo* imaging results for intracranially inoculated GL261/mchery-Luc glioma under the treatment of Py17XNL *Plasmodium* infection in combination with radiotherapy;
FIG. 21 is a graph showing survival curves of tumor-bearing mice intracranially inoculated with GL261/mchery-Luc glioma under the treatment of Py17XNL Plasmodium infection in combination with radiotherapy;
FIG. 22 is a graph showing weight growth curves of tumor-bearing mice intracranially inoculated with GL261/mchery-Luc glioma under the treatment of Py17XNL Plasmodium infection in combination with radiotherapy;
FIG. 23 is a graph showing *Plasmodium* infection rate curves for intracranially inoculated GL261/mchery-Luc glioma under the treatment of Py17XNL *Plasmodium* infection in combination with radiotherapy;
FIG. 24 is a graph showing CD3 immunohistochemistry results at the inoculation site of tumor-bearing mice intracranially inoculated with GL261/mchery-Luc glioma under the treatment of Py17XNL Plasmodium infection in combination with radiotherapy;
FIG. 25 is a graph showing LLC growth curves after re-inoculation of mice in the combination treatment group which were cured of intracranially inoculated with GL261/mchery-Luc glioma under the treatment of Py17XNL *Plasmodium* infection in combination with radiotherapy; and
FIG. 26 is a graph showing GL261/mcherry-Luc growth curves after re-inoculation of mice in the combination treatment group which were cured of intracranially inoculated with GL261/mchery-Luc glioma under the treatment of Py17XNL *Plasmodium* infection in combination with radiotherapy.

### DETAILED DESCRIPTION

Technical solutions of the present disclosure are further described below through examples. It is to be understood by those skilled in the art that the examples described below are used for a better understanding of the present disclosure and are not to be construed as specific limitations to the present disclosure.

### Example 1

### Experimental Case of Treatment of Subcutaneously Inoculated LLC Lung Cancer by Py17XNL Plasmodium Infection in Combination with Radiotherapy

### Experimental materials and reagents required in this example are as follows.

Animal: C57BL/6 mice, female, 6 to 8 weeks old, from SHANGHAI SLAC LABORATORY ANIMAL CO. LTD.; *Plasmodium*: mouse *Plasmodium yoelii* (*P. yoelii* 17XNL, MRA-593, Py), freely offered by Malaria Research and Reference Reagent Resource Center (MR4); mouse lung cancer cell line LLC: from the American Type Culture Collection (ATCC); Giemsa dye powder: purchased from Sigma-Aldrich; Avertin: 2,2,2-tribromoethanol, purchased from Sigma-Aldrich, T48402, formulated at a concentration of 1.2%; pathology-grade glass slide: Cat No. 10127101P-G, purchased from JIANGSU CITOTEST LABWARE MANUFACTURING CO., LTD.; cedar oil: from SHANGHAI BIAOBEN MOULD FACTORY, CHINA; PBS buffer: SH30256.01, purchased from Hyclone; and saline: purchased from CISEN PHARMACEUTICAL CO., LTD.

Red blood cell lysis buffer (ACK lysis buffer): formulated by NH₄Cl, 150 mM, KHCO₃, 10 mM, and Na₂EDTA, 0.1 mM.

FOXP₃ fixation/permeabilization concentrate: Mouse Foxp3 Fixation Concentrate (20x) (component 51-9006124), and Mouse Foxp3 Permeabilization Concentrate (5x) (component 51-9006125).

Antibody: FITC anti-mouse CD3 Antibody (Biolegend, Cat No: 100204), PerCP anti-mouse CD4 Antibody (Biolegend, Cat No: 100538), Brilliant Violet 510^{™} anti-mouse CD8a Antibody (Biolegend, Cat No: 100752), Brilliant Violet 711^{™} anti-mouse CD45 Antibody (Biolegend, Cat No: 103147), APC/Fire^{™} 750 anti-mouse/human CD11b Antibody (Biolegend, Cat No: 101262), Brilliant Violet 421^{™} anti-mouse Ly-6C Antibody (Biolegend, Cat No: 128032), PE/Cy7 anti-mouse Ly-6G Antibody (Biolegend, Cat No: 127618), APC anti-mouse CD25 Antibody (Biolegend, Cat No: 101910), PE anti-mouse FOXP3 Antibody (Biolegend, Cat No: 126404), APC Rat IgG2b, κ Isotype Ctrl Antibody (Biolegend, Cat No: 400611), PE Rat IgG2b, κ Isotype Ctrl Antibody (Biolegend, Cat No: 400608), Brilliant Violet 421^{™} Rat IgG2c, κ Isotype Ctrl Antibody (Biolegend, Cat No: 400725), PE/Cy7 Rat IgG2a, κ Isotype Ctrl Antibody (Biolegend, Cat No: 400521), TruStain FcX^{™} (anti-mouse CD16/32) Antibody (Biolegend, Cat No: 101320), and Zombie Yellow^{™} Fixable Viability Kit (Biolegend, Cat No: 423103).

Instrument: X-ray irradiator: Model No. RS2000, from Rad Source Technologies; lead block; microscope: Olympus microscope CX31; vernier caliper: electronic digital vernier caliper, Cat No. 678040, purchased from EXPLOIT TOOLS; and flow cytometer (Cytek^{®} Aurora).

### The specific operation steps are as follows.

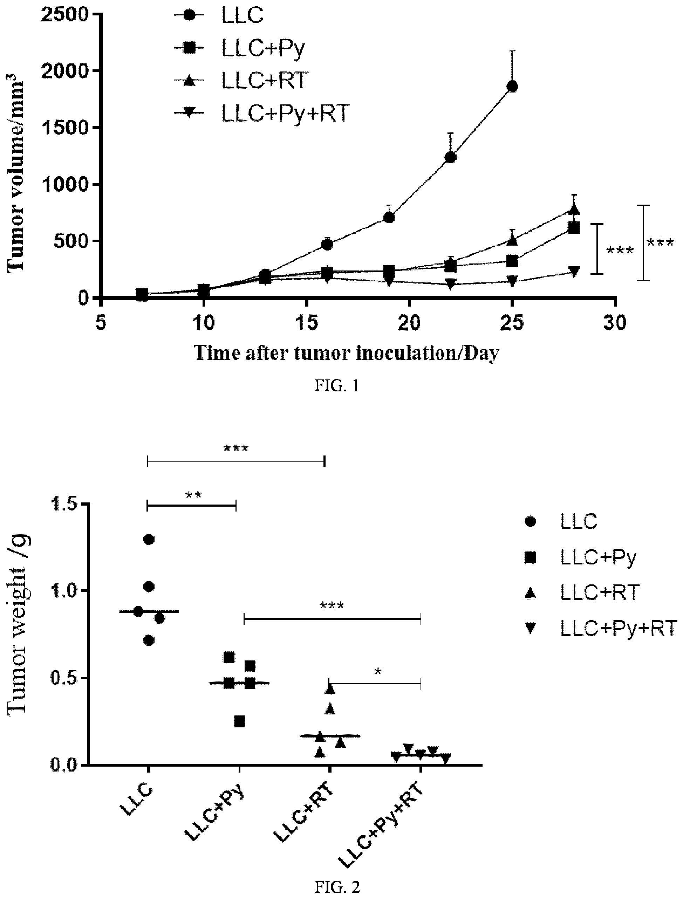

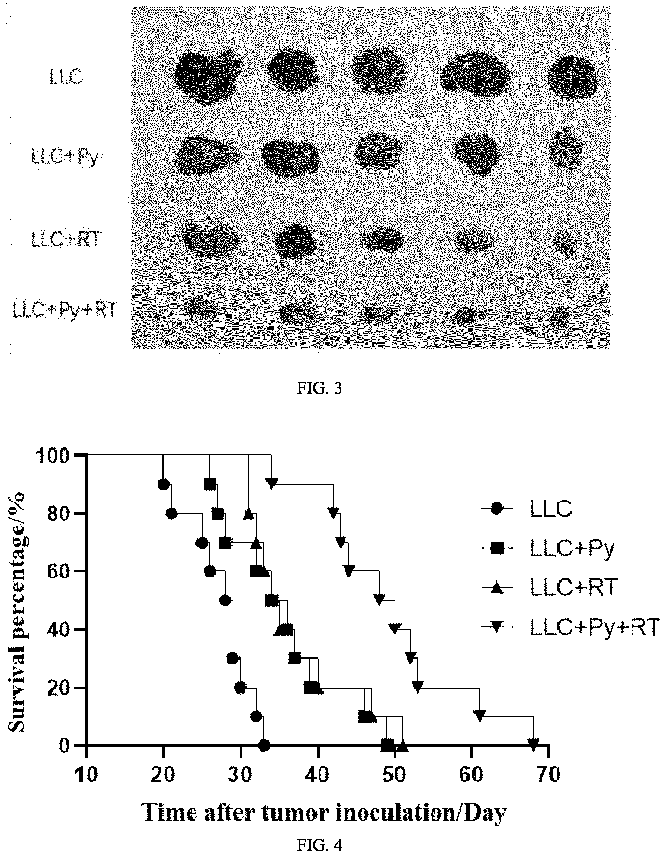

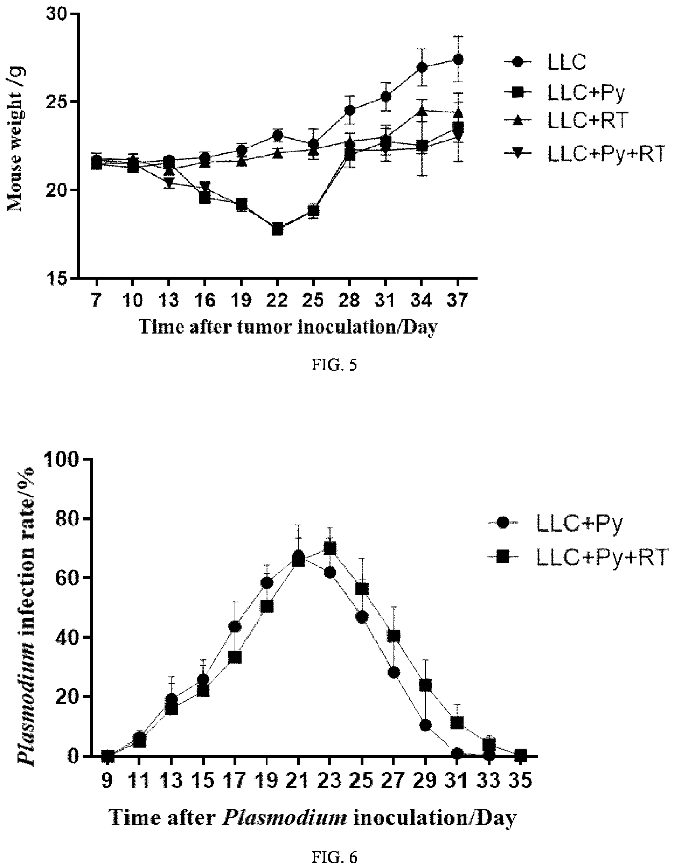

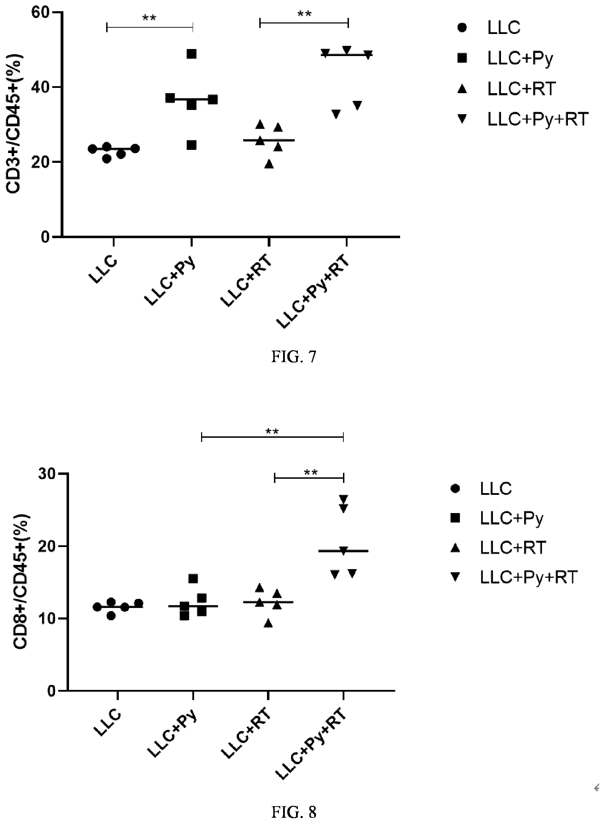

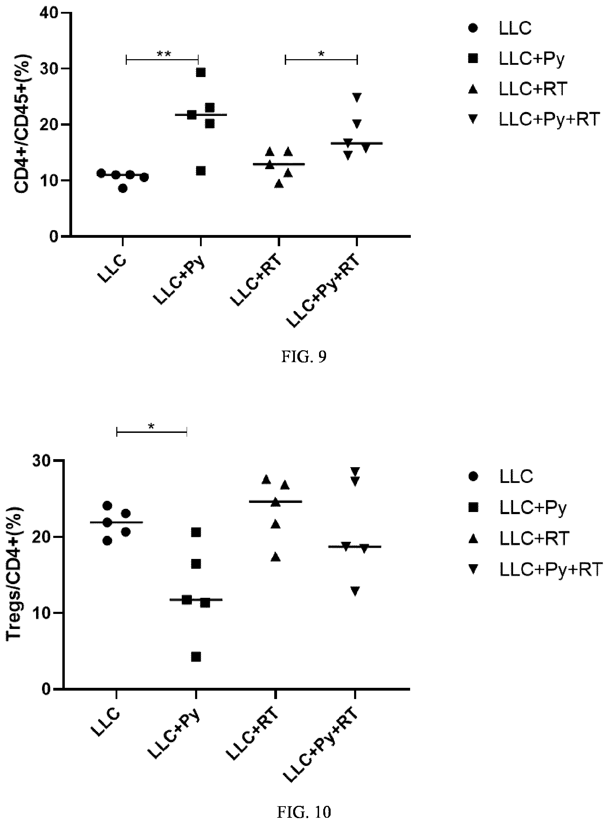

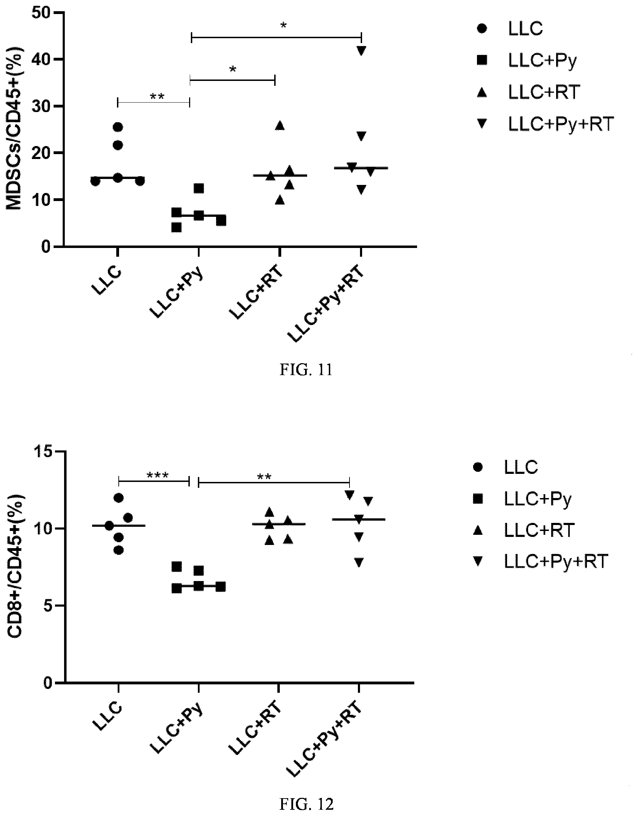

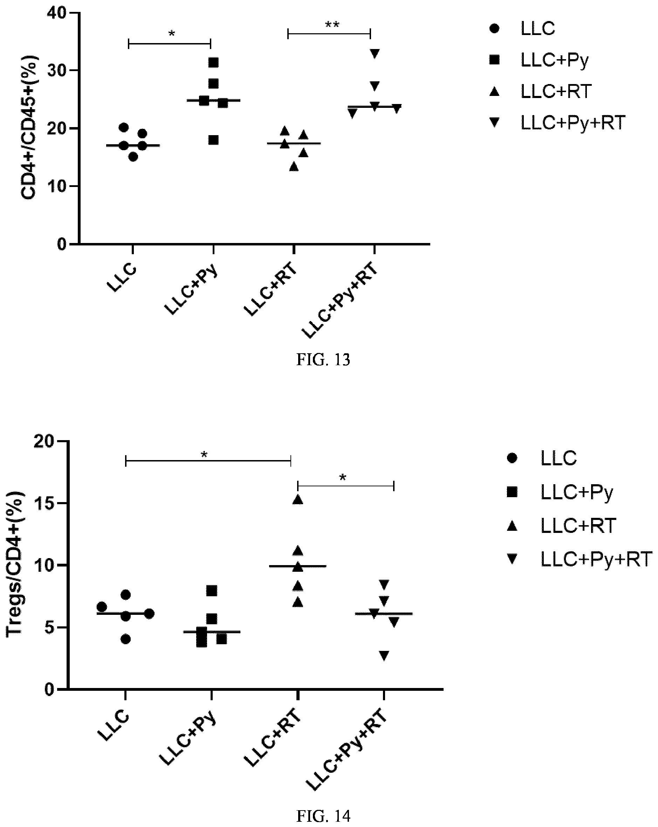

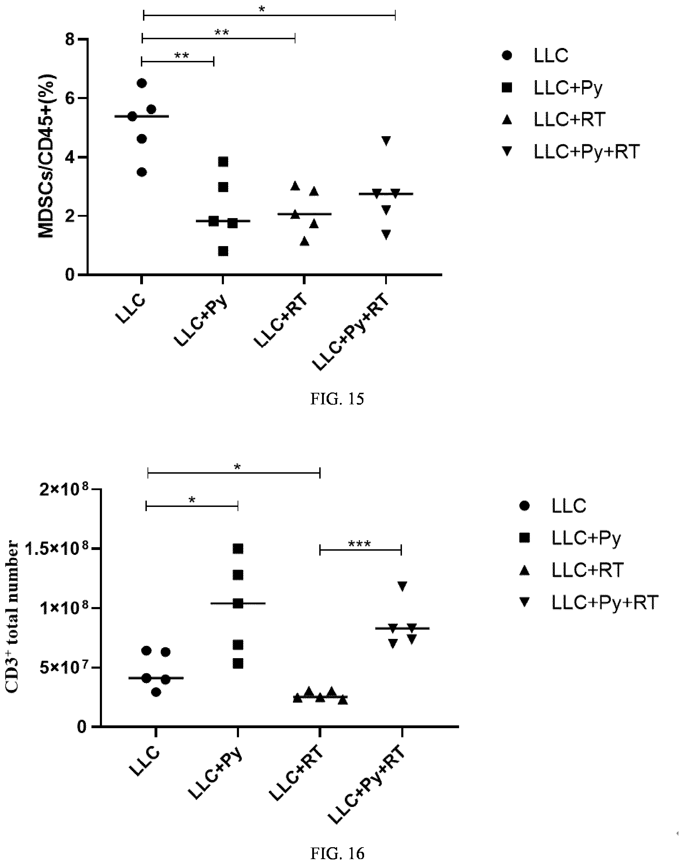

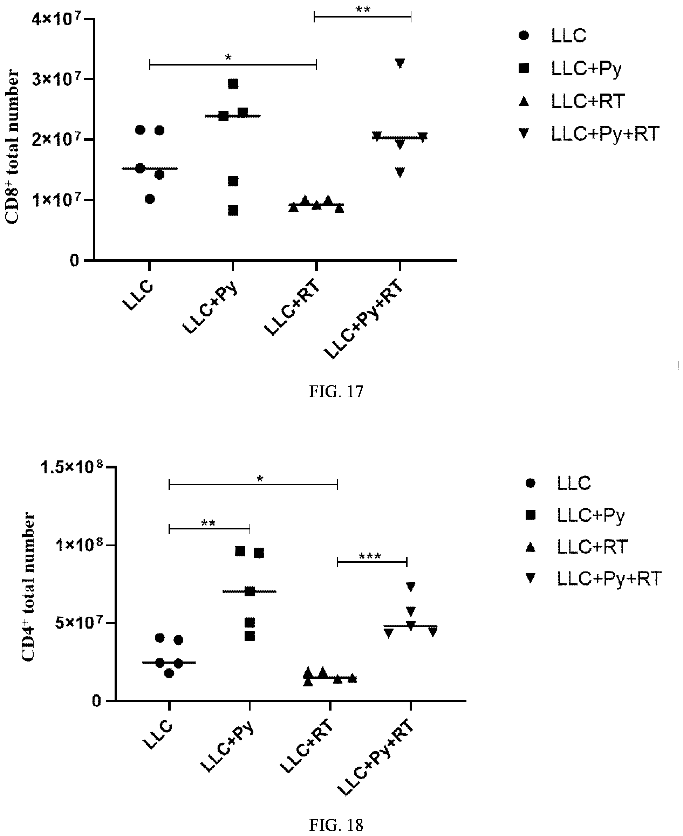

(I) An animal model was constructed in the following specific method:
   (1) Cell revival: The mouse lung cancer line LLC (Lewis lung carcinoma) was revived and incubated in an incubator with 5% CO₂ at a constant temperature of 37 °C.
   (2) Cell amplification culture: Cells were passaged every two days, and when the cells grew to 80% of the bottom of the petri dish, the cells were digested with 0.25% trypsin-EDTA digestion solution, diluted at a ratio of 1:3 and passaged.
   (3) Mouse shaving: C57BL/6 mice were shaved clean from the right scapula to the right back.
   (4) Single cell suspension preparation: The cells in the logarithmic growth phase were digested with 0.25% trypsin-EDTA digestive solution, washed with PBS three times and then resuspended with a serum-free 1640 medium, with a cell concentration of 5×10⁶.
   (5) Subcutaneous inoculation of tumor cells into mice: C57BL/6 mice were subcutaneously inoculated in the right scapular region, each injected with 0.1 mL of cell suspension with an inoculation amount of 5×10⁵ LLC cells per mouse, with the day of inoculation recorded as Day 0 of tumor inoculation.
   (6) Experimental grouping: On Day 7 after tumor inoculation, mice were grouped by stratified random sampling according to tumor size into four groups: tumor group (LLC), *Plasmodium yoelii* treatment group (LLC+Py), radiotherapy treatment group (LLC+RT), and combination treatment group (LLC+Py+RT), ten mice per group.
(II) Tumor-bearing mice were inoculated with *Plasmodium* in the following specific method:
   *(1) Plasmodium* revival: On Day 1 after LLC lung cancer inoculation, mouse *Plasmodium* blood (1.0 mL/vial) cryopreserved in a liquid nitrogen tank was quickly shaken in a 37 °C water bath, mixed and melted to keep the activity of *Plasmodium.*
   *(2) Plasmodium* inoculation: After the *Plasmodium* blood was mixed well, the *Plasmodium* blood was inoculated intraperitoneally into C57BL/6 mice, 0.2 mL per mouse, and two mice were inoculated each time as breeding mice.
   (3) Blood smear preparation and microscopy: About 1 to 1.5 µL of blood was collected from tails of mice, smeared on glass slides to prepare a long tongue-shaped blood smear with a length of 2.5 cm, and blow-dried with a blower. The blood smear was infiltrated with methanol for 1 min, stained with 1×Giemsa stain for 30 min, rinsed with tap water, and blow-dried. The *Plasmodium* infection rate was observed by a 100× oil immersion objective to observe the change in the *Plasmodium* infection rate.
   *(4) Plasmodium* solution preparation: When the infection rate reached 3% to 10%, the red blood cells (from one breeding mouse infected with *Plasmodium* and one Naive mouse not inoculated with *Plasmodium*) were counted. 5 µL of blood was collected from tails and resuspended in 995 µL of PBS, the red blood cells were counted, and the number of red blood cells infected with *Plasmodium* per mL of volume was calculated. An EP tube was moistened with 0.2 mL of 3.8% sodium citrate anticoagulant, the blood was collected using removing eyeball method, the required concentration and total amount of *Plasmodium* to be inoculated were calculated, and the *Plasmodium* was prepared into a concentration of 2.5×10⁶/mL with PBS.
   (5) Inoculation of tumor-bearing mice: On Day 7 after the tumor was subcutaneously inoculated, each mouse was inoculated with 0.2 mL of red blood cell solution, each mouse in the *Plasmodium yoelii* treatment group (LLC+Py) and the combination treatment group (LLC+Py+RT) was inoculated with the red blood cell solution containing 5×10⁵ *Plasmodia,* and each mouse in the control group was inoculated with the same number of red blood cells containing no *Plasmodium.*
(III) The mice were treated with radiotherapy in the following specific method:
   (1) On Day 10 after tumor inoculation, the mice in the radiotherapy treatment group and the combination treatment group were weighed and injected intraperitoneally with 1.2% Avertin, 0.4 mL per 20 g of body weight.
   (2) When the mice were anesthetized, the mice each were placed in a mouse cage and covered with a custom-made lead block, with the tumor tissue portion exposed.
   (3) The mouse cage was placed inside the irradiator, the probe was placed in the middle of the cage, and the door of the irradiator chamber was closed. "Manual" in the setup interface was clicked, the default values of the voltage and the current were set to 160 kV and 25 mA, the irradiation dose was set to 12 Gy, and the irradiation time required was calculated according to the radiation dose rate of 1.2 Gy/min.
   (4) The radiation dosimeter was calibrated to zero, and the radiation dose was re-measured; "START" was clicked, and the instrument started to execute the irradiation program.
   (5) After the mice were irradiated, the mice were taken out, and the radiation dose was calculated to make sure that the irradiation dose was the same for each mouse.
(IV) The flow cytometry detection was carried out in the following specific method:
   (1) On Day 19 after tumor inoculation, the mice were weighed, and the tumor size was measured and recorded.
   (2) Anticoagulant tubes were prepared, eyeballs were removed to collect blood, the mice were dissected, and then the spleens and the tumors were isolated and weighed.
   (3) Spleen cells isolation: spleen → stripping and crushing of the spleen → collection of the spleen cells and filtration → after centrifugation → lysis of red blood cells (the time was controlled within 2 minutes) → neutralization of lysis→ centrifugation → resuspension with 15 mL of medium → standing for 2 to 3 minutes, aspiration and transferred of 12 mL of supernatant to a 15 mL centrifuge tube → centrifugation → resuspension with 5 mL → cell counting (by a Trypan blue method) → collection of 2×10⁶ spleen cells → extracellular staining detection → CD3, CD4, CD8 and MDSCs, and collection of 3×10⁶ spleen cells → extracellular staining → fixation and permeabilization → FoxP3 staining → Tregs cell ratio detection.
   (4) Peripheral blood cell isolation: whole blood → centrifugation (350 g, 5 min) → collection of supernatant → cell washing → centrifugation (350 g, 5 min) → discarding of supernatant → red blood cell lysis → washing twice → resuspension with 1 mL of PBS (containing 2% FBS) → collection of 0.2 mL of cell suspension for detection → extracellular staining detection → CD3, CD4, CD8 and MDSCs, and collection of 0.3 mL of cell suspension → extracellular staining → fixation and permeabilization → FoxP3 staining → Treg cell ratio detection.
(V) The detection indicators include the following:
   (1) Tumor volume and size measurement: The tumor was measured every three days, and the tumor volume was calculated using the formula for calculating the volume of an ellipsoid (unit: cubic mm): (D×d×d)/2, wherein "D" denotes the long diameter of tumor, and "d" denotes the short diameter of tumor. The tumor size was represented by a mean tumor volume ± standard error of the mean (SEM), and the tumor growth curves were drawn. An intergroup statistical analysis was carried out by two-way analysis of variance (ANOVA), where "*" represented *P*≤0.05, and "**" represented *P*≤0.01, indicating that differences between groups were statistically significant.
   (2) Tumor weight comparison: On Day 19 after tumor inoculation, the tumors were separated, weighed with an electronic scale, and subjected to an unpaired Student's t-test, with the data as a mean tumor weight ± SD, where "*" represented *P*≤0.05, and "**" represented *P*≤0.01, indicating that differences between groups were statistically significant.
   (3) Mouse survival statistics: The survival was evaluated by the percentage of median survival and prolonged survival. The survival rate was estimated by the Kaplan-Meier method, the survival curve graph was drawn, and the median survival was calculated, where "*" represented *P*≤0.05, and "**" represented *P*≤0.01, indicating that differences between groups were statistically significant.
   *(4) Plasmodium* infection rate statistics: The *Plasmodium* infection rate was evaluated by a percentage of red blood cells infected with *Plasmodium* in mice, where the calculation formula was (number of red blood cells infected with *Plasmodiumltotal* number of red blood cells)×100%. The specific operations are as follows: blood was collected from tail veins, smeared on a glass slide, fixed with methanol, and stained with Giemsa stain. The number of red blood cells infected with *Plasmodium* and the total number of red blood cells were observed under a microscope. The total number of red blood cells was about 1000. The *Plasmodium* infection rate was calculated and represented by a mean infection rate ± standard error of the mean (SEM), and the *Plasmodium* infection cycle curves were drawn to observe whether radiotherapy had an effect on *Plasmodium* infection.
   (5) Mouse weight: The mice were weighed every three days, and the weight growth was represented by a mean weight ± standard error of the mean (SEM) to observe the effect of the *Plasmodium* infection on the weight of tumor-bearing mice.
   (6) Flow cytometry detection: The ratios of CD3⁺ T cells, CD4⁺ T cells, CD8⁺ T cells, MDSCs and Tregs in the spleen and the peripheral blood were detected, the absolute numbers of CD3⁺ T cells, CD4⁺ T cells and CD8⁺ T cells in the spleen were counted, and unpaired Student's t-tests were carried out, with the data as a mean tumor weight ± SD, where "*" represented *P*≤0.05, and "**" represented *P*≤0.01, indicating that differences between groups were statistically significant.
(VI) The detection results include the following:
   (1) As shown in the tumor growth curve in FIG. 1, the tumor weight comparison in FIG. 2 and the tumor anatomy in FIG. 3, compared with the growth of LLC lung cancer of the single *Plasmodium* infection treatment group and the single radiotherapy treatment group, the growth of LLC lung cancer in the combination treatment group where the *Plasmodium* infection in combination with radiotherapy was applied was significantly inhibited, which is statistically significant.
   (2) As shown in the survival statistics of tumor-bearing mice in FIG. 4, compared with the median survival (35 days) of the single *Plasmodium* infection treatment group and the median survival (34.5 days) of the single radiotherapy treatment group, the median survival of the tumor-bearing mice in the combination treatment group where the *Plasmodium* infection in combination with radiotherapy was applied was significantly prolonged, which is statistically significant.
   (3) As shown in FIG. 5, the weights of the tumor-bearing mice were less affected by radiotherapy, but the weights of the tumor-bearing mice in both the single *Plasmodium* infection treatment group and the combination treatment group decreased as the *Plasmodium* infection rate was raised and gradually increased as the *Plasmodium* infection rate was gradually decreased.
   (4) As shown in the *Plasmodium* infection rate curves in FIG. 6, the infection rate cycles of the single *Plasmodium* infection treatment group and the combination treatment group were the same, but the peak of *Plasmodium* infection in the combination treatment group was delayed by three days compared with that of the single *Plasmodium* infection treatment group.
   (5) As shown in the immune indicators of the peripheral blood in FIGS. 7 to 11, the *Plasmodium* infection increased the T cells ratios of CD3⁺/CD45⁺ and CD4⁺/CD45⁺ in the peripheral blood, radiotherapy did not increase the T cells ratios of CD3⁺/CD45⁺, CD8⁺/CD45⁺ and CD4⁺/CD45⁺, and the *Plasmodium* infection decreased the T cells ratios of Tregs/CD4⁺ T and MDSCs/CD45⁺ in the peripheral blood. The *Plasmodium* could enhance anti-tumor immunity by increasing the T cells ratios of CD3⁺/CD45⁺, CD8⁺/CD45⁺ and CD4⁺/CD45⁺ and decreasing the T cells ratios of Tregs/CD4⁺ T and MDSCs/CD45⁺ in the peripheral blood of tumor-bearing mice.
   (6) As shown in the immune indicators of the spleen in FIGS. 12 to 18, the *Plasmodium* infection increased the T cells ratios of CD3⁺/CD45⁺ and CD4⁺/CD45⁺ in the spleen, decreased the T cells ratio of CD8⁺/CD45⁺ and increased the absolute numbers of CD3⁺ T cells, CD4⁺ T cells and CD8⁺ T cells in the spleen, and the *Plasmodium* infection decreased the T cells ratios of Tregs/CD4⁺ T and MDSCs/CD45⁺. The radiotherapy did not change the T cells ratios of CD3⁺/CD45⁺, CD8⁺/CD45⁺ and CD4⁺/CD45⁺ in the spleen, increased the ratio of Tregs/CD4⁺ T cells in the spleen, and decreased the absolute numbers of CD3⁺ T cells, CD8⁺ T cells and CD4⁺ T cells in the spleen. The *Plasmodium* could enhance anti-tumor immunity by increasing the T cells ratios of CD3⁺/CD45⁺ and CD4⁺/CD45⁺, decreasing the T cells ratios of Tregs/CD4⁺ cells and MDSCs/CD45⁺ and increasing the absolute numbers of CD3⁺ T cells, CD8⁺ T cells and CD4⁺ T cells in the spleen of the tumor-bearing mice, whereas radiotherapy increased the ratio of Tregs/CD4⁺ T cells in the spleen, decreased the absolute numbers of CD3⁺ T cells, CD8⁺ T cells and CD4⁺ T cells in the spleen and thus impaired anti-tumor immunity. The *Plasmodium* infection could form complementary anti-tumor effects with radiotherapy.

### Example 2

### Experimental Case of Treatment of Intracranially Inoculated GL261 glioma in situ by Plasmodium Infection in Combination with Radiotherapy

### Experimental materials and reagents required in this example include the following:

Animal: C57BL/6 mice, female, 6 to 8 weeks old, from SHANGHAI SLAC LABORATORY ANIMAL CO. LTD.; tumor cell: GL261 from GUANGZHOU CELLCOOK BIOTECH CO., LTD., and GL261/mcherry-Luciferase constructed by GUANGZHOU CAS LAWVAC BIOTECH CO., LTD.; *Plasmodium*: mouse *Plasmodium yoelii* (*P. yoelii* 17XNL, MRA-593, Py), freely offered by Malaria Research and Reference Reagent Resource Center (MR4); luciferase substrate: D-Luciferin, Potassium Salt D-fluorescein potassium salt, purchased from SHANGHAI YEASEN BIOTECHNOLOGY CO., LTD.; Giemsa dye powder: purchased from Sigma-Aldrich; DMEM basic medium: 11995065, purchased from Gibco; FBS fetal bovine serum: 04-001-1ACS, purchased from Biological Industries; Avertin: 2,2,2-tribromoethanol, purchased from Sigma-Aldrich, T48402, formulated at a concentration of 1.2%; alcohol; iodine tincture; hydrogen peroxide; saline; silicone oil; gentamicin sulfate injection; and erythromycin ointment.

CD3 antibody: ab16669, purchased from ABCAM; HRP secondary antibody: ab97051, purchased from ABCAM; and hematoxylin: 51275-100 mL, purchased from SIGMA. Instrument: X-ray irradiator: Model No. RS2000, from Rad Source Technologies; lead block; *in vivo* imager: IVIS Spectrum, purchased from PERKINELMER; brain stereotaxic instrument: purchased from SHENZHEN RWD LIFE SCIENCE CO., LTD.; hand-held cranial drill: purchased from SHENZHEN RWD LIFE SCIENCE CO., LTD.; microsyringe: in the specification of 5 µL, purchased from HAMILTON; electronic digital vernier caliper: Cat No. 678040, purchased from EXPLOIT TOOLS; and surgical instruments: surgical pads, timers, surgical blades, scalpel handles, ophthalmic scissors, ophthalmic surgical straight tweezers, ophthalmic surgical curved tweezers, needle holders, sewing needles, cotton swabs, sutures, etc.

### The specific operation steps are as follows.

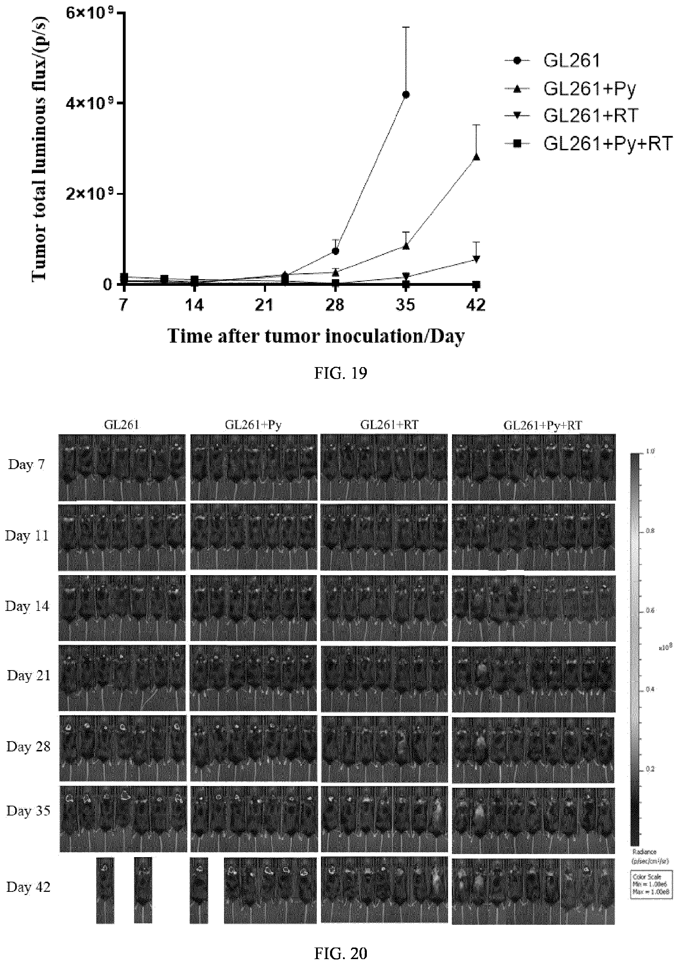

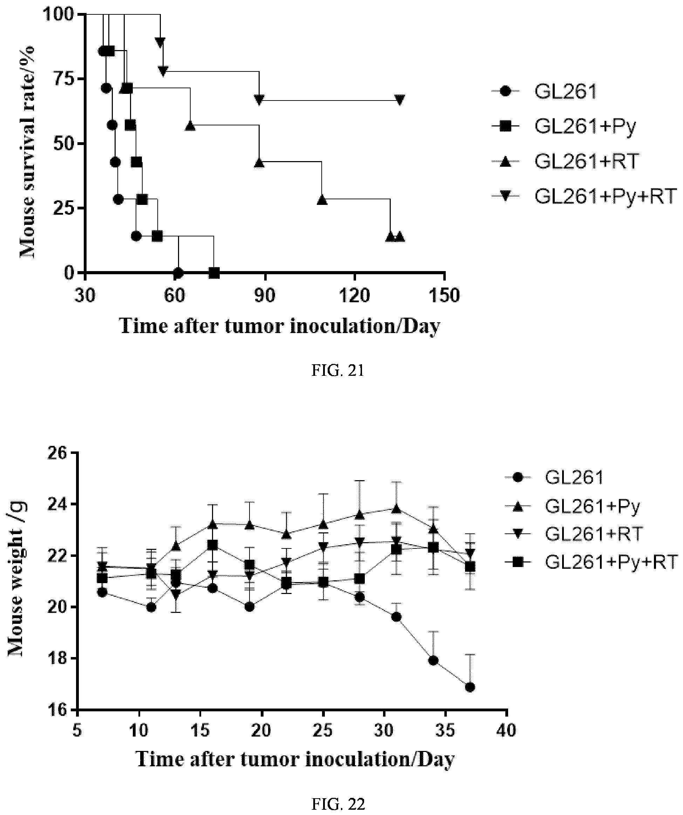

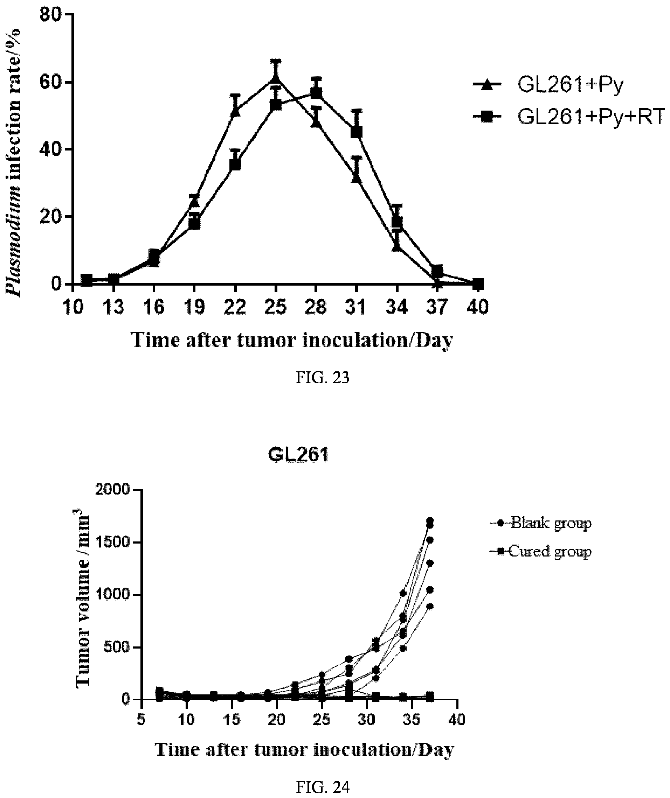

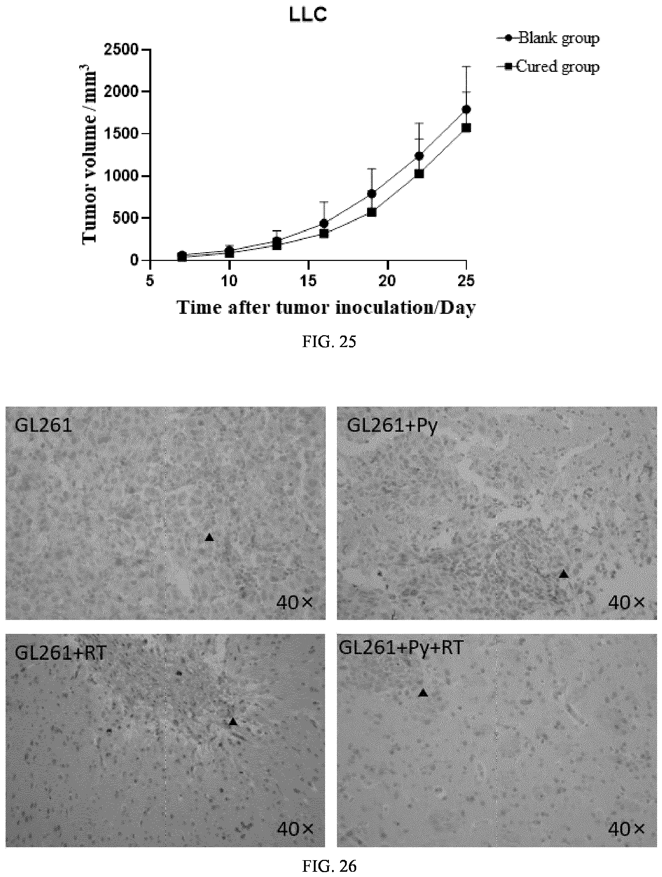

(I) An animal model was constructed in the following specific method:
   (1) Cell revival: The mouse glioma cell line GL261/mcherry-Luc was revived and incubated in an incubator with 5% CO₂ at a constant temperature of 37 °C.
   (2) Cell amplification culture: Cells were passaged every two days, and when the cells grew to 80% of the bottom of the petri dish, the cells were digested with 0.25% trypsin-EDTA digestion solution, diluted at a ratio of 1:3 and passaged.
   (3) GL261/mcherry-luc cell preparation: GL261/mcherry-luc cells in the logarithmic growth phase were washed with PBS, digested with trypsin, washed with PBS 3 times, counted, resuspended with an RPIM 1640 medium, with a cell concentration of 2×10⁸/µL, and stored on ice.
   (4) The surgical instruments were disinfected with 75% alcohol.
   (5) Preparation of injection needles and microsyringes: An injection needle and a connection hose were filled with silicone oil with a 1 mL syringe to check whether there were blockages in the hose to prevent air bubbles, the syringe was replaced with a microsyringe, and the microsyringe was installed with the injection needle and drew up an appropriate number of cells to check whether there was air leakage.
   (6) Mouse anesthesia: 1.2% Avertin anesthetic was administered intraperitoneally at a dose of 0.4 mL/20 g of body weight to anesthetize the mice.
   (7) Mouse shaving: The hair on the top of the head was shaved clean with a shaving knife, with the hair between eyes and ears shaved as clean as possible.
   (8) The mice each were fixed onto the brain stereotaxic instrument, the teeth of the mice were fixed first (the incisors were put on the opening of the nose clamp), the mouth was slightly opened with tweezers to pull out the tongue, then the back of the head was fixed (the ear bars were put in both ears mainly to fix the cheekbones), and finally, the nose clamp was fixed tightly.
   (9) The skin was disinfected with iodine tincture and cut with a scalpel along the midline, the cut was approximately 10 mm from the position behind the eyes to the ears, the connective tissue on the skull was scraped with the assistance of the scalpel, and the skull was wiped with cotton swabs dipped with hydrogen peroxide to make the anterior fontanel of the skull clearer.
   (10) The tip of the injection needle aimed at the anterior fontanelle, with the anterior fontanelle as the reference point, the transverse direction of the head of the each mouse as the X-axis and the longitudinal midline as the Y-axis, the X-axis and Y-axis of the stereotaxic instrument were set to 0 mm, the injection needle was moved, with a position 1 mm ahead the X-axis and 1.8 mm right to the midline of the Y-axis as the drilling position, the injection needle was lowered and aimed at the surface of the skull of each mouse, the Z-axis was set to 0 mm, the injection needle was raised, and the drilling position on the skull was marked with a pencil, with the hole diameter of about 2 mm.
   (11) Cell injection: The injection needle was fixed at the position of the anterior fontanel, the injection position (1.8 mm right, 1 mm ahead and 3.4 mm deep) was set using an XYZ digital display instrument, the cells were injected with the injection volume of 1 µL for about 1 minutes, and the needle was retained for 5 min and then slowly withdrawn.
   (12) Suture: After the cut was sutured, erythromycin ointment was applied to the cut, and then 0.1 mL of gentamicin sulfate injection was injected intramuscularly.
   (13) *In vivo* imaging: On Day 7 after tumor inoculation, the mice were imaged *in vivo,* and the total luminous flux of the tumor site in the head of the mice was calculated.
   (14) Experimental grouping: The mice were grouped by stratified random sampling into four groups: glioma control group (GL261), *Plasmodium yoelii* treatment group (GL261+Py), radiotherapy treatment group (GL261+RT), and combination treatment group (GL261+Py+RT).
(II) The mice were inoculated with *Plasmodium* in the following specific method:
   (I) *Plasmodium* revival: On Day 1 after tumor inoculation, mouse *Plasmodium* blood (1.0 mL/vial) cryopreserved in a liquid nitrogen tank was quickly shaken in a 37 °C water bath, mixed and melted to keep the activity of *Plasmodium.*
   (2) *Plasmodium* inoculation: After the *Plasmodium* was revived and mixed well, the *Plasmodium* blood was inoculated intraperitoneally into C57BL/6 mice with 0.2 mL/mouse, and two mice were inoculated each time.
   (3) Blood smear preparation and microscopy: On Day 4 and Day 6 after the breeding mice were inoculated with *Plasmodium,* about 1 to 1.5 µL of blood was collected from tails of mice, smeared on glass slides to prepare a long tongue-shaped blood smear with a length of 2.5 cm, and blow-dried with a blower. The blood smear was infiltrated with methanol for 1 min, stained with 1×Giemsa stain for 30 min, rinsed with tap water, and blow-dried. The *Plasmodium* infection rate was observed by a 100× oil immersion objective to observe the change in the *Plasmodium* infection rate.
   (4) *Plasmodium* solution preparation: When the infection rate reached 3% to 10%, the red blood cells were counted. 5 µL of blood was collected from tails and resuspended in 995 µL of PBS, the red blood cells were counted, and the number of red blood cells infected with *Plasmodium* per mL of volume was calculated. An EP tube was moistened with 0.2 mL of 3.8% sodium citrate anticoagulant, the blood was collected using removing eyeballs method, the required concentration and total amount of *Plasmodium* to be inoculated were calculated, and the *Plasmodium* was prepared into a concentration of 2.5×10⁶/mL with PBS.
   (5) Inoculation of tumor-bearing mice: On Day 7 after tumor inoculation, each mouse was inoculated with 0.2 mL of red blood cell solution, that is, each mouse was inoculated with 5×10⁵ *Plasmodia,* and each mouse in the control group was inoculated with the same number of red blood cells containing no *Plasmodium.*
(III) The mice were treated with radiotherapy in the following specific method:
   (1) On Day 10 after tumor inoculation, the mice in the radiotherapy treatment group and the combination treatment group were weighed and injected intraperitoneally with Avertin, 0.4 mL per 20 g of body weight.
   (2) When the mice each were anesthetized, the mice each were placed in a mouse cage and covered with a custom-made lead block, with the tumor inoculation site on the head exposed while protecting other parts of the mice, such as the eyes.
   (3) The mouse cage was placed inside the irradiator, the probe was placed in the middle of the cage, and the door of the irradiator chamber was closed. "Manual" in the setup interface was clicked, the default values of the voltage and the current were set to 160 kV and 25 mA, the irradiation dose was set to 12 Gy, and the irradiation time required was calculated according to the radiation dose rate of 1.2 Gy/min.
   (4) The radiation dosimeter was calibrated to zero, and the radiation dose was re-measured; "START" was clicked, and the instrument started to execute the irradiation program.
   (5) After the mice were irradiated, the mice were taken out, and the radiation dose was calculated.
(IV) Immunohistochemical detection was carried out to detect the CD3⁺ T cell infiltration in gliomas in the following specific method:
   (1) On Day 21 after tumor inoculation, the mice were weighed and recorded.
   (2) Sampling: Anticoagulant tubes were prepared, eyeballs were removed to collect blood, the mice were dissected, and then the spleen and the brain were isolated.
   (3) The brain tissue was fixed with 10% neutral formaldehyde.
   (4) Trimming: The part of tissue needed for the experiment was trimmed into a block of 0.5×0.5×0.2 cm and put in the embedding cassette, and if the tissue was too large, the tissue was fixed for half an hour after trimmed.
   (5) Washing: The trimmed tissue was rinsed with running water for 2 h to wash away the excess fixation liquid.
   (6) Dehydration: The tissue was dehydrated using 70% alcohol for 1 h, 85% alcohol for 1 h, 95% alcohol for 1 h, anhydrous ethanol for 45 min and anhydrous ethanol for 45 min.
   (7) Transparency: The tissue was immersed in the mixed liquid mixed by 100% alcohol and xylene equally by volume for 15 min and immersed in xylene for 0.5 h (or until the tissue was transparent), during which xylene must be changed once. Because ethanol and paraffin are not soluble and xylene is soluble in both ethanol and paraffin, after dehydration, the tissue needs to be immersed in xylene for transition. When all the tissue was occupied by xylene, the light could pass through, and the tissue showed different degrees of transparency.
   (8) Paraffin impregnation: The tissue was impregnated in paraffin wax which was melted at 60 °C, and paraffin impregnation was carried out in the oven at 60 °C using three jars of paraffin wax, 1 h for each jar.
   (9) Embedding: Dissolved paraffin wax was poured into the embedding frame to form a film, the tissue block was quickly placed with the section side facing down, after the position was set right, the tissue block was placed into the embedding cassette, and liquid wax was added dropwise and waited to be solidified.
   (10) Sectioning: A tissue slice with a thickness of 4 to 6 µm was sectioned and fished up on a slide-resistant siliconized slide, and the slide was baked in the oven at 60 °C for 3 h and then stored in the refrigerator at 4 °C.
   (11) Deparaffinization: Before the experiment, the slide was baked in the oven at 60 °C for 30 min. The paraffin slice was placed in fresh xylene and immersed three times, each time for 10 min.
   (12) Hydration: After excess liquid was removed, the slice was placed in anhydrous ethanol and immersed three times, each time for 3 min. With excess liquid removed, the slice was placed in 95% ethanol and immersed twice, each time for 3 min. With excess liquid removed, the slice was placed in 75% ethanol and immersed twice, each time for 3 min. Then the slice was rinsed with distilled water for 1 min and placed in the PBS buffer.
   (13) Endogenous peroxidase blocking: 3% H₂O₂ was added dropwise to the slide, and the slide was allowed to stand at room temperature for 10 min. The slide was rinsed with the PBS buffer three times, each time for 3 min.
   (14) Antigen retrieval: Antigen retrieval was carried out with 0.01 M citrate buffer at pH of 6.0 in a high-pressure heating method, and then rinsed with the PBS buffer three times, each time for 3 min.
   (15) Blocking: The slice was blocked with non-immune normal serum blocking buffer at room temperature for 20 min, and after that, excess liquid was shaken off.
   (16) Primary antibody incubation: According to the size of the tissue, 100 µL or an appropriate amount of primary antibody was added dropwise, and then incubated at 37 °C for 60 min and rinsed with the PBS buffer three times, each time for 3 min.
   (17) Secondary antibody incubation: 100 µL or an appropriate amount of enzyme-labeled goat anti-rabbit IgG polymer was added dropwise, and then incubated at room temperature for 20 min and rinsed with the PBS buffer three times, each time for 3 min.
   (18) Color development: An appropriate amount of freshly prepared DAB color development solution was added, and incubated at 25 °C for 8 min.
   (19) Counterstaining: The slice was rinsed with tap water for 10 min and incubated with hematoxylin staining solution, where the incubation time was increased or shortened depending on the coloring effect of the solution, and then the slice was rinsed with tap water for 15 min.
   (20) Differentiation and bluing: The slice was differentiated with 1% hydrochloric alcohol for 5 s and rinsed with running water 10 min, and if the stained color was very light, the slice could continue to be stained with hematoxylin.
   (21) The slice was dehydrated, made transparent, sealed, examined under a microscope, and photographed.
(V) Secondary tumor inoculation was carried out in the following specific method:
   (1) Cell revival: The LLC lung cancer and GL261 glioma cells were revived.
   (2) Mouse shaving: Cured mice in the combination treatment group and blank mice were shaved clean on the left and right of their bodies.
   (3) Cell inoculation: The cured mice in the combination treatment group and the blank mice each were inoculated with 5×10⁵ LLC cells on their left bodies and with 2×10⁶ GL261 cells on their right bodies.
   (4) Tumor measurement: The long and short diameters of the tumor were measured every three days, the size of the tumor was calculated, and the tumor growth curves were drawn.
(VI) The detection indicators include the following:
   *(1) In vivo* imaging of mice: *In vivo* imaging was carried out on the mice every seven days, and the total luminous flux of the brain was calculated to characterize the size of the brain tumor. The tumor size was represented by a mean tumor volume ± standard error of the mean (SEM), and the tumor growth curves were drawn. An intergroup statistical analysis was carried out by two-way ANOVA, where "*" represented p≤0.05, and "**" represented p≤0.01, indicating that differences between groups were statistically significant.
   (2) Mouse survival statistics: The survival was evaluated by the percentage of median survival and prolonged survival. The survival rate was estimated by the Kaplan-Meier method, the survival curve graph was drawn, and the median survival was calculated, where "*" represented *P*≤0.05, and "**" represented *P*≤0.01, indicating that differences between groups were statistically significant.
   *(3) Plasmodium* infection rate statistics: The *Plasmodium* infection rate was evaluated by a percentage of red blood cells infected with *Plasmodium* in mice, where the calculation formula was (number of red blood cells infected with *Plasmodiumltotal* number of red blood cells)× 100%. The specific operations are as follows: blood was collected from tail veins, smeared on a glass slide, fixed with methanol, and stained with Giemsa stain. The number of red blood cells infected with *Plasmodium* and the total number of red blood cells were observed under a microscope. The total number of red blood cells was about 1000. The *Plasmodium* infection rate was calculated and represented by a mean infection rate ± standard error of the mean (SEM), and the *Plasmodium* infection cycle curves were drawn to observe whether radiotherapy had an effect on *Plasmodium* infection.
   (4) Mouse weight: The mice were weighed every three days, and the weight growth was represented by a mean weight ± standard error of the mean (SEM) to observe the effect of the *Plasmodium* infection on the weight of tumor-bearing mice.
   (5) Brain tissue immunohistochemistry: The infiltration of CD3⁺ T cells in the brain tumor and areas adjacent to the tumor was observed on Day 21 after GL261 cell inoculation.
(VII) The detection results include the following:
   (1) As shown the luminous flux statistics of glioma obtained by *in vivo* imaging in FIG. 19 and the *in vivo* imaging results in FIG. 20, compared with the growth of glioma GL261 of the single *Plasmodium* infection treatment group and the single radiotherapy treatment group, the growth of glioma GL261 intracranially inoculated in mice *in situ* in the combination treatment group where the *Plasmodium* infection in combination with radiotherapy was applied was significantly inhibited.
   (2) As shown in the survival curves of the tumor-bearing mice in FIG. 21, compared with the survival of the single *Plasmodium* infection treatment group and the single radiotherapy treatment group, the survival of the tumor-bearing mice intracranially inoculated *in situ* in the combination treatment group where the *Plasmodium* infection in combination with radiotherapy was applied was significantly prolonged.
   (3) As shown in the weight growth curves of the tumor-bearing mice in FIG. 22, the weights of the tumor-bearing mice were less affected by the *Plasmodium* infection and radiotherapy, and the weights of the tumor-bearing mice in the model groups gradually decreased due to the rapid growth of gliomas in the brain.
   (4) As shown in the infection rate curves in FIG. 23, the infection rate cycles of the single *Plasmodium* infection treatment group and the combination treatment group were the same, but the peak of *Plasmodium* infection in the combination treatment group was delayed by three days compared with that of the single *Plasmodium* infection treatment group.
   (5) As shown in CD3 immunohistochemistry at the brain tumor site in FIG. 24 (the arrows in the figure indicate CD3⁺ T-expressing cells), the *Plasmodium* infection, the radiotherapy, and the combination treatment all could significantly inhibited the growth of brain tumors and promoted the infiltration of CD3⁺ T cells into the brain tumors and the paracancerous areas. Compared with the single *Plasmodium* infection treatment and the single radiotherapy, the *Plasmodium* infection in combination with radiotherapy prompted more CD3⁺ T cell infiltration.
   (6) As shown in the tumor growth curves of re-inoculated GL261 in FIG. 25 and re-inoculated LLC in FIG. 26, the growth of re-inoculated GL261 gliomas in the mice cured by the *Plasmodium* infection in combination with radiotherapy was inhibited, whereas no effective anti-tumor effect was developed on non-homologous LLC lung cancer, indicating that the *Plasmodium* infection in combination with radiotherapy could form effective immune memory.

The applicant has stated that although the use of *Plasmodium* in the preparation of an anti-tumor preparation for use in combination with radiotherapy of the present disclosure is described through the examples described above, the present disclosure is not limited to the examples described above, which means that implementation of the present disclosure does not necessarily depend on the examples described above. It is to be apparent to those skilled in the art that any improvements made to the present disclosure, equivalent replacements of raw materials of the product, additions of adjuvant ingredients, selections of specific methods, etc. in the present disclosure all fall within the protection scope and the disclosure scope of the present disclosure.

Although the preferred embodiments of the present disclosure are described above in detail, the present disclosure is not limited to details in the embodiments described above, and various simple modifications can be made to the technical solutions of the present disclosure without departing from the technical concept of the present disclosure. These simple modifications are all within the protection scope of the present disclosure.

Additionally, it is to be noted that if not in collision, the specific technical features described in the embodiments described above may be combined in any suitable manner. In order to avoid unnecessary repetition, various possible combination manners are no longer described in the present disclosure.

## Claims

1. Use of *Plasmodium* in preparation of an anti-tumor preparation for use in combination with radiotherapy.

2. The use of claim 1, wherein the *Plasmodium* comprises any one or a combination of at least two of *Plasmodium falciparum, Plasmodium vivax, Plasmodium malariae, Plasmodium ovale* or *Plasmodium knowlesi,* and preferably is *Plasmodium vivax.*

3. The use of claim 1 or 2, wherein an inoculation amount of the *Plasmodium* is not less than 100 *Plasmodia* or not less than five *Plasmodium* sporozoites.

4. The use of any one of claims 1 to 3, wherein the radiotherapy comprises an alpha ray, a beta ray, a gamma ray, an X ray, an electron beam or a proton beam;
preferably, the radiotherapy comprises a single radiotherapy, a multiple radiotherapy or a fractionation radiotherapy;
preferably, the radiotherapy comprises a systemic radiotherapy or a local radiotherapy.

5. The use of any one of claims 1 to 4, wherein the tumor comprises a lung cancer, a gastric cancer, a colon cancer, a liver cancer, a breast cancer, a prostate cancer, an ovarian cancer, a pancreatic cancer, and a brain tumor.

6. The use of any one of claims 1 to 5, wherein a dosage form of the preparation comprises any one of pharmaceutically acceptable dosage forms;
preferably, the preparation further comprises any one or a combination of at least two of pharmaceutically acceptable pharmaceutical adjuvants;
preferably, the *Plasmodium* is a *Plasmodium* carried on a pharmaceutical carrier;
preferably, the pharmaceutical carrier comprises a liposome, a micelle, a dendrimer, a microsphere or a microcapsule.

7. An anti-tumor preparation, wherein the anti-tumor preparation is a *Plasmodium* preparation for use in combination with radiotherapy.

8. The anti-tumor preparation of claim 7, wherein the preparation and the radiotherapy are administered concurrently;
preferably, the preparation and the radiotherapy are administered sequentially;
preferably, the *Plasmodium* comprises any one or a combination of at least two of *Plasmodium falciparum, Plasmodium vivax, Plasmodium malariae, Plasmodium ovale* or *Plasmodium knowlesi,* and preferably is *Plasmodium vivax,*
preferably, an inoculation amount of the Plasmodium is not less than 100 Plasmodia or not less than five Plasmodium sporozoites;
preferably, the tumor comprises a lung cancer, a gastric cancer, a colon cancer, a liver cancer, a breast cancer, a prostate cancer, an ovarian cancer, a pancreatic cancer, and a brain tumor;
preferably, an administration manner of the preparation comprises intravenous injection, intraperitoneal injection, intramuscular injection, subcutaneous injection, oral administration, sublingual administration, nasal administration or percutaneous administration, and preferably is intravenous injection.

9. An anti-tumor combination therapy, wherein the combination therapy is a combination of radiotherapy and a *Plasmodium* therapy.

10. The combination therapy of claim 9, wherein the radiotherapy and the *Plasmodium* are administered concurrently;
preferably, the radiotherapy and the Plasmodium are administered sequentially;
preferably, the *Plasmodium* comprises any one or a combination of at least two of *Plasmodium falciparum, Plasmodium vivax, Plasmodium malariae, Plasmodium ovale* or *Plasmodium knowlesi* and preferably is *Plasmodium vivax,*
preferably, an inoculation amount of the *Plasmodium* is not less than 100 *Plasmodia* or not less than five *Plasmodium* sporozoites;
preferably, the tumor comprises a lung cancer, a gastric cancer, a colon cancer, a liver cancer, a breast cancer, a prostate cancer, an ovarian cancer, a pancreatic cancer, and a brain tumor;
preferably, an administration manner of the *Plasmodium* comprises intravenous injection, intraperitoneal injection, intramuscular injection, subcutaneous injection, oral administration, sublingual administration, nasal administration or percutaneous administration, and preferably is intravenous injection.
